# EUROPEAN PATENT SPECIFICATION

(11) **EP 1 803 470 B1**
(45) Date of publication and mention of the grant of the patent: **29.04.2009**
(21) Application number: 06256602.1
(22) Date of filing: 28.12.2006
(51) Int. Cl.: A61L 2/18, A61L 2/24

(54) **Storage enclosure for sanitising/sterilising endoscopes**
Lagerbehälter zum Reinigen/Sterilisieren von Endoskopen
Récipient de stockage pour nettoyer/stériliser des endoscopes

(30) Priority: 29.12.2005 US 321244
(43) Date of publication of application: 04.07.2007
(73) Proprietor: ETHICON, INC., Somerville, New Jersey 08876 (US)
(72) Inventor: Lin, Szu-Min, Irvine, CA 92620 (US); Platt, Robert C. Jr., Laguna Niguel, CA 92677 (US); Mirchandani, Vinod, San Ramon, CA 94582 (US)
(74) Representative: Fisher, Adrian John

(56) References cited:
- EP-A1- 1 016 371
- WO-A-01/56459
- WO-A-01/56615
- WO-A-20/05056060
- DE-C1- 3 819 257
- US-A- 5 405 587
- US-A- 6 068 815

## Description

### Background of the Invention

The present invention relates to endoscope washing and decontamination.

Devices exist for washing and decontaminating endoscopes automatically. They are typically termed automated endoscope reprocessors (AER). One such device is shown in U.S. Published Patent Application No. 2004/0118413 published June 24, 2004, (the '413 application). Typical AER will comprise a basin into which the endoscope is coiled and into which flows solution for cleaning and disinfection or sterilization. Individual connections are typically made to the various connectors on the endoscope to provide fluid under pressure to those channels for washing and disinfection. At the end of the procedure the endoscope is removed from the basin. Accordingly, even if the endoscope where completely sterilized in the procedure, its removal from the basin would break that sterility.

A system has been devised in which an endoscope is coiled into a cassette, which cassette is then placed into the basin for processing. When the cassette is removed from the AER the endoscope maintains its sterility within the cassette. Please see U.S. Patent No. 5,534,221. However, such a cassette is not suitable for long-term storage of most endoscopes. Due to the delicate nature of their internal structure, it can be harmful to leave them coiled for extended periods of time.

DE-C1-38 19 257 discloses an endoscope enclosure of the type set forth in the preamble of the accompanying claim 1.

US 6 068 815 discloses an endoscope processor comprising sealable cassettes with ports for sterilant supply, pumping means, an operational housing. The endoscope is fixed on a bay by clamping members

EP 1 016 371 discloses an endoscope processor comprising sealable cassettes with ports for sterilant supply, pumping means, an operational housing for internal/external cleaning/sterilisation.

WO 2005/056060 discloses an endoscope processor comprising sealable cassettes with ports for sterilant supply, pumping means, an operational housing. The cassettes are hermitically sealed and divided up into several compartments.

WO 01/56615 discloses an endoscope sterilization enclosure comprising connectors, a movable support, a hinged door.

### Summary of the Invention

According to a first aspect of the invention, there is provided an endoscope enclosure for sterilizing an endoscope as set forth in the accompanying claim 1.

Further, optional, aspects are set out in the dependent claims.

### Brief Description of the Drawings

FIG. 1 is a cut-away view of an endoscope (prior art) which can be processed in the present invention;
FIG. 2 is a front perspective view of an endoscope processor according to the present invention;
FIG. 3 is a sectional view taken along lines 3 - - 3 of FIG. 2;
FIG. 4 is a side perspective view of the endoscope processor of FIG. 2, showing one of the cabinets moved out;
FIG. 5 is a detailed cut-away view of an endoscope within one of the cabinets of the endoscope processor of FIG. 2;
FIG. 6 is a cut-away view of a portion of a channel connector for use in the endoscope processor of FIG. 2;
FIG. 7 is a cut-away view of an alternative channel connector;
FIG. 8 is a cut-away view of a supporting surface within one of the cabinets of the endoscope processor of FIG. 2;
FIG. 8a is a cut-away view of an alternative supporting surface; and
FIG. 9 is a cut-away view of a further alternative supporting surface.

### Detailed Description of the Drawings

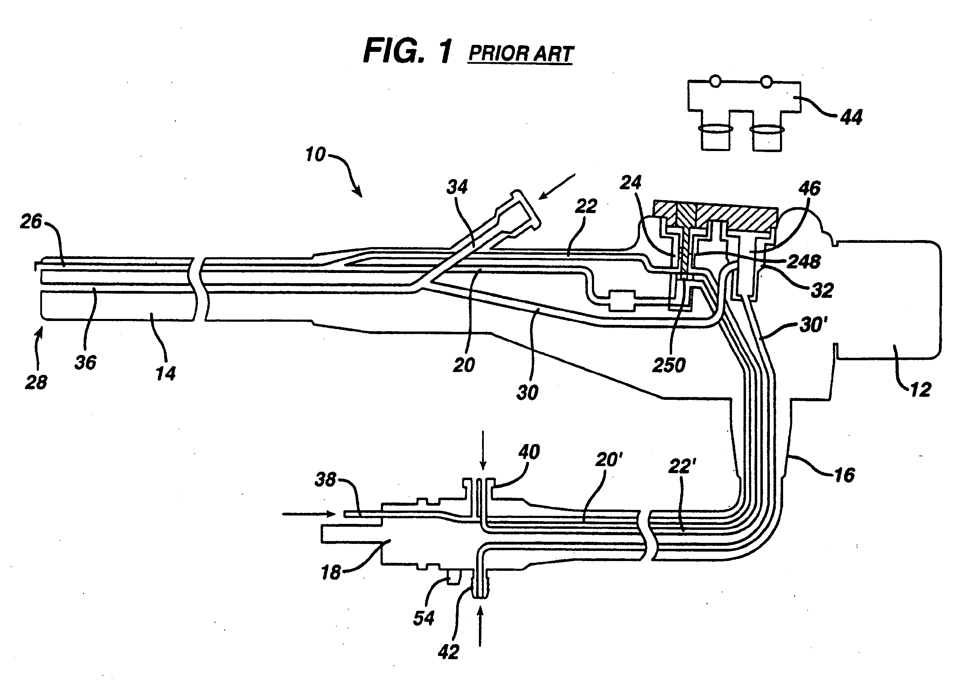

FIG. 1 shows an endoscope 10 having a control head 12 and a flexible insertion tube 14 extending there from. An umbilical cord 16 connects the control head 12 to a light housing 18. An air channel 20 and a water channel 22 intersect a first cavity 24 in the control head 12. They extend from the first cavity 24 down the insertion tube 14 intersecting to form a combined air and water channel 26 which extends to a distal end 28 of the insertion tube. A suction channel 30 extends from a second cavity 32 in the control head 12 down the insertion tube and intersects with a biopsy or instrument channel 34 to form a combined suction/biopsy channel 36 which extends to the distal end 28.

The channels extend also from the control head 12 to the light housing 18 through the umbilical cord 16 and in that section will be given their numeral designator with the addition of a prime symbol. The air channel 20' extends from the first cavity 24 to terminate at a port 38 in the light housing 18. The water channel 22' extends from the first cavity 24 to terminate at a port 40 in the light housing 18. The suction channel 30' extends from the second cavity 32 to terminate in a port 42 in the light housing 18.

A valve mechanism 44 fits within the first and second cavities 24 and 32 to control flow of air, water and suction during operation of the endoscope 10. During cleaning and sterilization the valve mechanism is removed and placed into a cage (not shown in FIG. 1) for processing with the endoscope 10. A channel separator 46 is inserted into the first and second cavities 24 and 32 to isolate the air channel 20 from the water channel 22 and to enclose the first and second cavities 24 and 32.

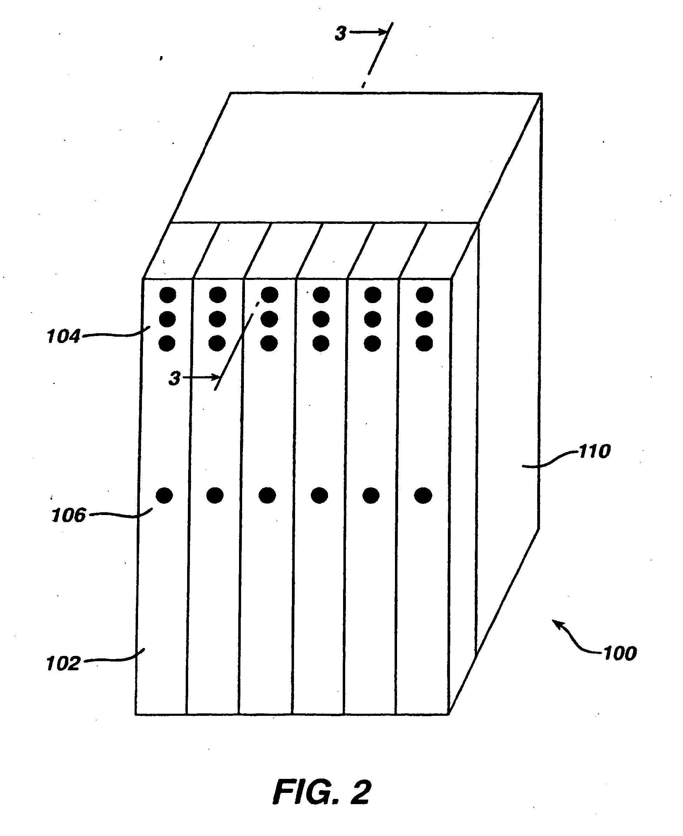

Turning also now to FIG. 2, an endoscope processor system 100 is shown. It comprises a plurality of independent cabinets 102, each adapted for processing an endoscope 10. Status lights 104 or other indicators are preferably provided on each cabinet 102 to show the status of whether the cabinet is loaded with an endoscope 10, and whether processing has been completed and completed successfully, as well as other information which might be useful to an operator. Handles 106 and castors 108 are provided for easy maneuvering of the cabinets 102.

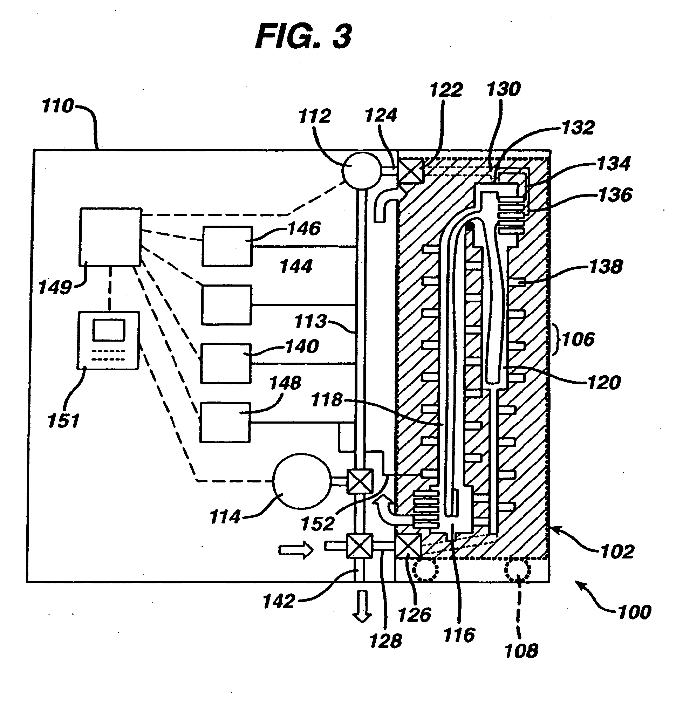

Turning also now to FIG. 3, the cabinet 102 connects to a main housing 110 which contains a fluid pump 112 and vacuum pump 114 and associated circulation plumbing 113. Interior of the cabinet 102 is an endoscope receiving space 116 having a first depending cavity 118 for receiving the endoscope umbilical cord 16 and a second depending cavity 120 for receiving the endoscope insertion tube 14. Preferably, the endoscope will fit closely within the receiving space 116. Many endoscopes have flexible tubing over a meter long so the cabinet 102 should be of sufficient height to accommodate them. A supply connection 122 on the cabinet 102 connects to an outlet connection 124 on the housing 110 and a return connection 126 on the cabinet 102 connects to an inlet connection 128 on the housing 110. Each of the supply connection 122 and return connection 126 are preferably of the self closing type such that when disconnected from the housing 110 they remain closed leaving the receiving space 116 sealed.

A supply manifold 130 leads from the supply connection to a valve 132 feeding the interior space 116 and to a valve 134 feeding a channel connector 136. The channel connector 136 supplies fluid to each of the channels in the endoscope 10 and will be described in more detail later. Alternatively, multiple connections between the cabinet 102 and the housing 110 can be made so that each channel in the endoscope 10 can be fed by an individual supply line, preferably each controlled by a constant volume pump in the control housing and drawing flow from the circulating plumbing 113. Examples of such plumbing in an endoscope reprocessor are known to those of skill in the art and include the '413 application. The supply manifold further supplies liquid to a plurality of nozzles 138 lining the receiving space 116. These nozzles 138 enhance the ability to flow liquid over the endoscope 110 and thereby enhance the cleaning action, but if sufficient flow is achieved through the first and second depending cavities 118 and 120 the nozzles 138 can be omitted.

Cleaning and sterilization of an endoscope are achieved by first flowing a cleaning liquid, including a detergent and/or other cleaning agents supplied to the flow by a detergent dispensing system 140. It flows through the channel connector 136 into each of the endoscope channels (i.e. the air channels 20 and 20', water channels 22 and 22', suction channels 30 and 30' and the biopsy channel 34), through the valve 132 into the interior space 116 and through the first and second depending cavities 118 and 120, and through the nozzles 138. It drains from the bottom of the first and second depending cavities 118 and 120 and returns to the pump 112 through the return connection 126. The system is then drained through a drain 142 and fresh filtered rinse water supplied from a water supply system 144. The rinse water flows similarly and is drained. A supply of fresh filtered water is added along with a sterilant such as orthophthalaldehye, gluteraldehyde, hydrogen peroxide or peracetic acid, from a sterilant supply system 146. After the sterilant solution has been circulated for a sufficient time to effect the desired level of disinfection or sterilization it is drained a rinse performed with fresh filtered rinse water. An alcohol rinse could follow. Preferably, clean filtered air from an air supply system 148 is blown through the system to dry the endoscope 10, its channels, and the receiving space 116. Preferably, a controller 149, having a display and input device 151, controls such a cycle. The cycle is described in general terms only; other features as may be known to those of skill in the art may be incorporated therein, such as processes for checking the integrity of connections, checking for channel blockages etc.

The vacuum pump 114 can be employed to enhance the cycle. After cleaning and exposure to a liquid sterilant which is vaporizable to produce a vapor sterilant, such as hydrogen peroxide or peracetic acid, the vacuum pump 114 can be employed to lower pressure within the receiving space 116 to vaporize the liquid sterilant thereby drying the endoscope 10 and exposing it to a sterilizing vapor. Temperature, pressure, peroxide amount and concentration, and pump down rate affect the overall efficacy. A pressure of about 667 to 1333 N/m²(5 to 10 torr) and a temperature of about 30° to 45° C are desirable. Details of such a process can be found in US Patent Nos. 5,851,485 and US 6,030,579. Especially if pumping to lower pressures, it may be desirable to provide separate lines and connections from the vacuum pump 114 and the cabinet 102 along with the ability to close the other connections such as at 124 and 128 thereby simplifying the seals needed to maintain a vacuum in the receiving space 116.

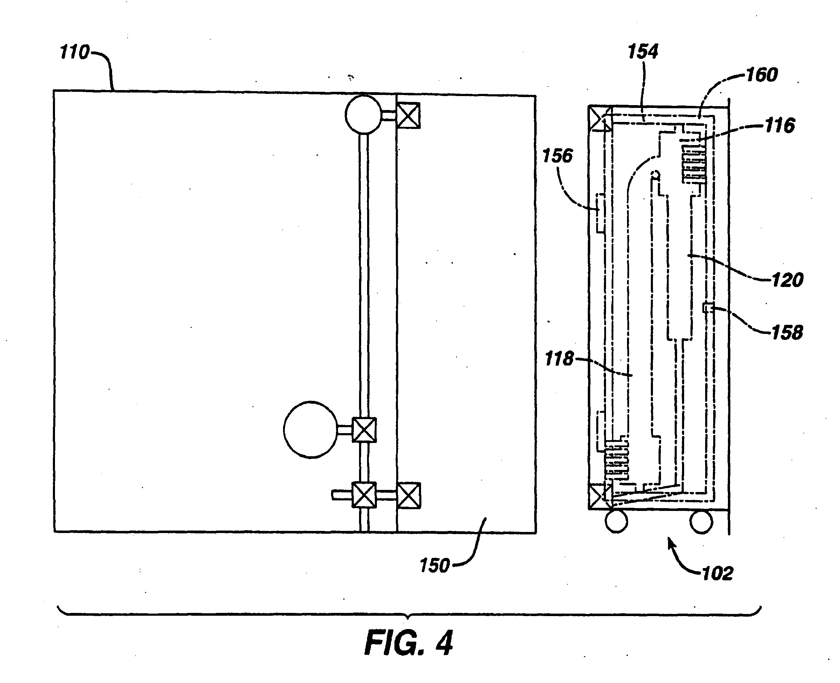

Turning also now to FIG. 4, the cabinet 102 moves on castors 108 from a position disposed exterior of an open compartment 150 (FIG. 4) to a position interior thereof (FIG. 3) wherein the supply connection 122 mates with the outlet connection 124, the return connection 126 mates with the inlet connection 128 and other electrical and fluid connections are made. Preferably the cabinet 102 contains sensors for temperature and pressure and also electrical connections to operate the valves 132 and 134 and the lights 104 as well as other electrical devices as may be desired therein. An air supply connection 152 can be provided for testing the integrity of the endoscope 10 sheath via a port which leads to a space within the endoscope internal of the sheath.

The endoscope 10 can be loaded into the receiving space 116 through a side door 154 having hinges 156 and a latch 158. A seal 160 around the door 154 prevents liquids from the washing and liquid sterilant portions of the cycle from leaking out, and prevents air from infiltrating in during a vacuum portion of the cycle if such is employed. It also maintains sterility of the endoscope 10 after a sterilization process be preventing ingress of potentially contaminating microorganisms.

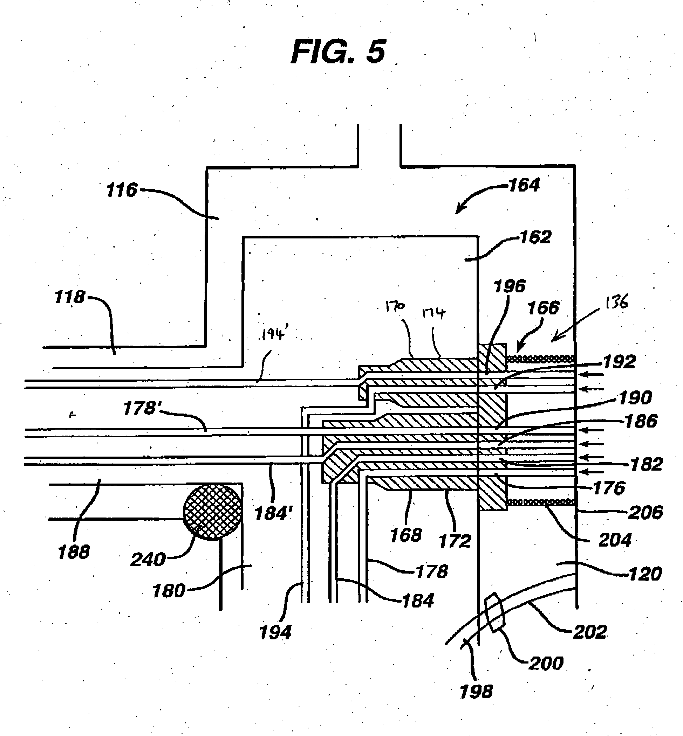

Turning also now to FIG. 5, the channel connector 136 is shown in greater detail fitted within a control head 162 of an endoscope 164. (Please note that while the structure of the endoscope 164 differs slightly from the endoscope 10 in placement of the channels it otherwise corresponds and similar terminology is used in its description. The present invention is intended for general use and each endoscope will have its own structure and channel arrangement.) It comprises a body 166 adapted to fit closely within first and second cavities 168 and 170 of the control head 162 and having a plurality of channels therethrough. A first body portion 172 fits within the first cavity 168 and a second body portion 174 fits within the second cavity 170. A first channel 176 passes through the first body portion 172 to intersect with a water channel 178 in an insertion tube 180 of the endoscope 164. A second channel 182 passes through the first body portion 172 to intersect with an air channel 184. A third channel 186 passes through the first body portion 172 to intersect with an umbilical cord 188 portion of the air channel 184' (note that the umbilical cord 184 portions of the channels are designated with a prime). A fourth channel 190 passes through the first body portion 172 to intersect with the water channel 178'. A fifth channel 192 passes through the second body portion 174 to intersect with a suction channel 194 and a sixth channel 196 passes through the second body portion 174 to intersect with the suction channel 194'.

Each of the first through sixth channels 176, 182, 186, 190, 192 and 196 connect either directly or through intermediate tubing to the supply manifold 130 downstream of the valve 134. Preferably, rather than a single supply manifold 130 individual lines and connections are provided on the cabinet 102 connecting to individual pumps in the housing 110 for each of these channels in the channel connector 136. Also, in addition to these connections, most endoscopes have a separate biopsy channel 198 and associated connector 200 an additional connection, such as with a connection tubing 202 as is known in the art. A non-occluding connection such as taught in pending US Patent Application No. 11/141431 in which the connector has flaps which move away from the connection surface under certain flow conditions, such as high flow, to limit occlusions are preferred.

Reduction of occlusions during the process is to be desired. If the channel connector 136 were to be moved inwardly and outwardly at points during the cycle, the areas in which it contacts the first and second cavities 168 and 170 would be contacted with cleaning and sterilization fluid. Mechanical means can be provided to impart such movement, such as a motor and linkage connected thereto. However, it is preferred to limit the complexity as there is a desire to keep the cost of the cabinet 102 to a minimum as it is employed for storage as well as for cleaning and sterilization and a typical user might desire a separate cabinet for each of their endoscopes.

To economize the function of reducing occlusion via channel connector 136 movement it is desirable to employ the energy contained within the flowing fluids to effect such movement, thereby negating the requirement for additional expensive equipment. Springs 204 between a surface 206 forming a portion of the receiving space 116 and the channel connector 136 urge the channel connector 136 inwardly of the first and second cavities 168 and 170. Extra flow through its channels provides pressure tending to urge the channel connector 136 outwardly. Thus, by controlling flow through the channels the position of the channel connector can be changed.

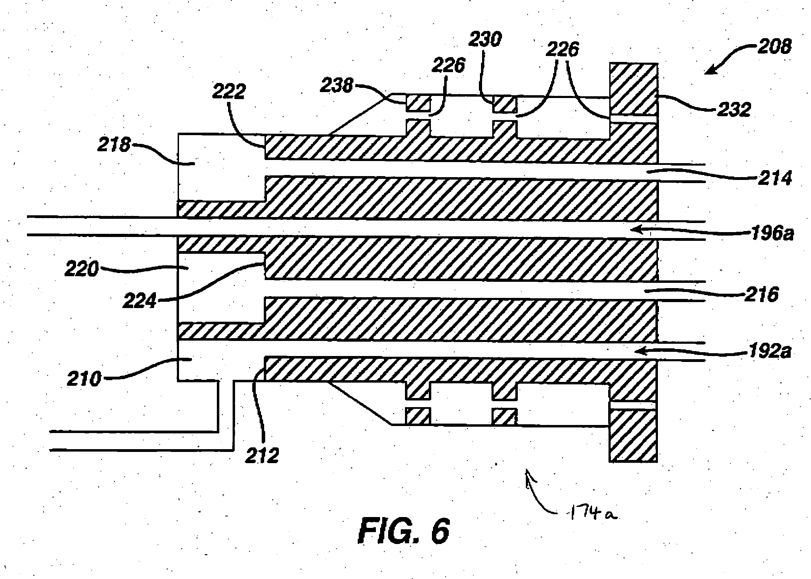

The structure of the channel connector 136 can enhance this ability. Turning also to FIG. 6, which shows a first embodiment 208 of the second body portion 174a (it is the lesser in complexity) of the channel connector 136. Parts herein which are generic and described before, will be designated with a following character "a." The fifth channel 192a terminates in a space 210 having a surface 212 whereupon flow therethrough increases pressure on the surface 212 to urge the second body portion 174a outwardly of the second cavity 170. Separate channels 214 and 216 terminate in spaces 218 and 220 with surfaces 222 and 224 whereupon flow into the spaces 218 and 220 would tend to urge the second body portion 174a outwardly. Openings 226 through annular positioning flanges 228, 230 and an outer body portion 232 allow outward movement without a suction blockage.

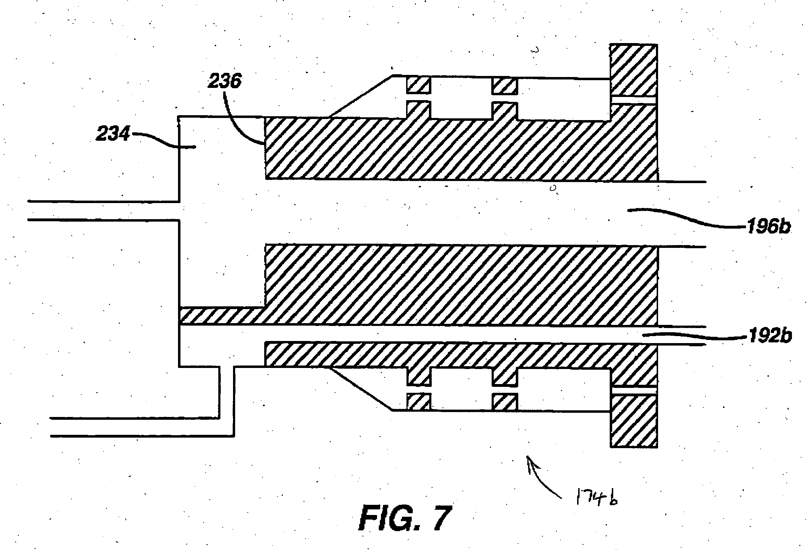

Turning also to FIG. 7, an alternative approach, with sub-labels "b" employs rather than separate channels 214 and 216, a channel 196b which opens into a space 234 with a surface 236 which allows flow therethrough to create a pressure which urges the second body portion 174b outwardly. A regular washing flow is not sufficient to overcome the force of the springs 204, whereas an increased flow creates a pressure sufficient to overcome the force of the springs 204 and move the body portion 174b outwardly.

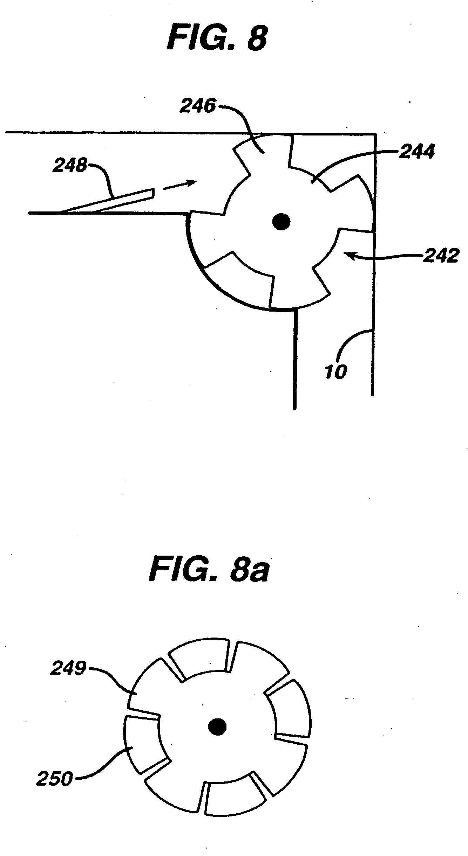

Turning also to FIG. 8, another source of occlusion occurs between the endoscope 10 and a contact surface 240 (FIG. 3) between the first depending cavity 118 and second depending cavity 120. In other locations the movement of the fluid therethrough should prevent continuous occlusion, but this location bears the weight of the endoscope 10 and despite fluid flow thereby the endoscope 10 may not move with respect to this contact surface 240.

To alleviate issue with such occlusion, means such as a rotating contact surface 242 can be employed. The rotating contact surface 242 comprises a wheel 244 having blades 246 thereon which is urged into rotation via a jet 248 connected to the supply manifold 130 and aimed at the blades 246. A plurality of axially aligned wheels 249 and 250 rotating, preferably in opposite directions, (FIG. 8a) can also be employed so as to limit the movement of the endoscope 10 engendered by the wheels 249 and 250. Motors or other means could also be employed rather than the jet(s) 248 to effect such movement.

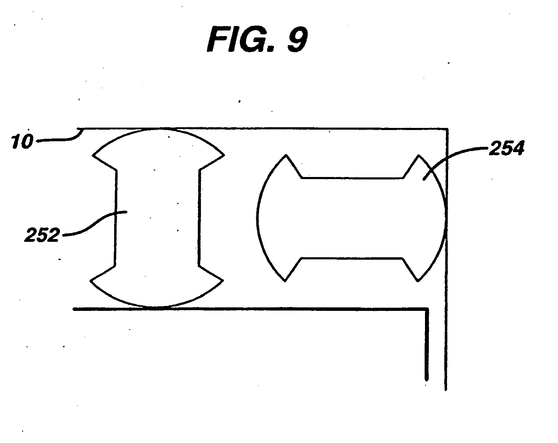

Turning also to FIG. 9, first and second rotating cams 252 and 254 move the endoscope upwards and outwards respectively. They would preferably be motor driven. In addition to reducing occlusion, they would also be able to move the endoscope 10 with respect to the channel connector 136 if it were rigidly attached to the cabinet 102.

Additional disclosure on channel connectors can be found in US Application Serial No. 11/263,010.

The invention has been described with reference to the preferred embodiments. Obviously, modifications and alterations will occur to others upon reading and understanding the preceding detailed description. It is intended that the invention be construed as including all such modifications and alterations insofar as they come within the scope of the appended claims or the equivalents thereof.

## Claims

1. An endoscope enclosure (102) for sterilizing an endoscope (10) having a body (12), and first and second flexible tubes (14, 16) attached to the body, the endoscope enclosure comprising:
a receiving space (116) adapted to receive such an endoscope (10) in an orientation with the first flexible tube (14) depending vertically downwardly from the endoscope body (12), wherein the receiving space (116) comprises a first downwardly depending space (118) adapted to receive the first flexible tube (14) of the endoscope depending downwardly, and a second downwardly depending space (120), separated from the first downwardly depending space (118) by a supporting surface (240), and adapted to receive the second flexible tube (16) of the endoscope depending downwardly;
an inlet fluid connector (122), fluidly communicating with the receiving space (116) and which has a closure for isolating the receiving space from an exterior environment through the inlet fluid connector; and
an outlet fluid connector (126), fluidly communicating with the receiving space (116) and which has a closure for isolating the receiving space from the exterior environment through the outlet fluid connector;
the receiving space (116) being sealed from the exterior environment whereupon such an endoscope can be sterilized by passing fluid through the receiving space (116) via the inlet and outlet fluid connectors (122, 126) and stored therein in an orientation with the first and second flexible tubes (14, 16) depending downwardly, and in sterile form being protected from ingress of potentially contaminating microorganisms,
**characterised in that** the endoscope enclosure (102) further comprises movement means adapted to move the supporting surface (240; 242) during a sterilization procedure whereby to reduce occlusions, and **in that** the supporting surface (242) is adapted to rotate.

2. An endoscope enclosure according to claim 1, further comprising means adapted to cause liquid to flow under pressure and thereby to rotate the supporting surface (240; 242).

3. An endoscope enclosure according to claim 1 or 2, wherein the enclosure (102) further comprises a series of connections (136) for supplying liquid to one or more lumens in the endoscope (10).

4. An endoscope enclosure according to any preceding claim, further comprising a hinged door (154) leading into the receiving space (116) whereby to insert such an endoscope (10) and a seal (160) thereabout.

5. An endoscope enclosure according to claim 4, further comprising a second hinged door (154) on an opposite side of the enclosure leading into the receiving space (116) whereby to insert the endoscope (10) and a seal (160) thereabout.

6. An endoscope enclosure according to any preceding claim, further comprising castors (108) on a lower portion thereof to aid in moving the enclosure (102).

7. An endoscope enclosure according to any preceding claim, wherein the receiving space (116) is sufficiently long to accommodate an endoscope with a first flexible tube (14) having a length of one meter.

8. An endoscope enclosure according to claim 7, wherein the receiving space (116) is sufficiently long to accommodate an endoscope with a first flexible tube (14) having a length of two meters.

9. An endoscope enclosure (102) according to any preceding claim, in combination with an endoscope (10) having a body (12), and first and second flexible tubes (14, 16) attached to the body, wherein the receiving space (116) is sized and shaped to closely fit the size and shape of the endoscope (10).

## Patentansprüche

1. Endoskopbehälter (102) zum Sterilisieren eines Endoskopes (10) mit einem Körper (12) und einer ersten und einer zweiten flexiblen Röhre (12, 16), die an dem Körper angebracht sind, wobei der Endoskopbehälter Folgendes umfasst:
einen Aufnahmeraum (116) der dafür eingerichtet ist, ein solches Endoskop (10) in einer Lage aufzunehmen, in welcher die erste flexible Röhre (14) von dem Endoskopkörper (12) vertikal nach unten hängt, wobei der Aufnahmeraum (116) einen ersten nach unten hängenden Raum (118) umfasst, der dafür eingerichtet ist, die erste flexible Röhre (14) des Endoskops nach unten hängend aufzunehmen, und einen zweiten nach unten hängenden Raum (120), der von dem ersten nach unten hängenden Raum (118) durch eine Trägerfläche (240) beabstandet ist und dafür eingerichtet ist, die zweite flexible Röhre (16) des Endoskops nach unten hängend aufzunehmen;
einen Fluideinlassanschluss (122), der in Fluidverbindung mit dem Aufnahmeraum (116) steht und der einen Verschluss zum Isolieren eines Aufnahmeraums von einer Außenumgebung durch den Fluideinlassanschluss aufweist; und
einen Fluidauslassanschluss (126), der in Fluidverbindung mit dem Aufnahmeraum (116) steht und der einen Verschluss zum Isolieren des Aufnahmeraums von der Außenumgebung durch den Fluidauslassanschluss aufweist;
wobei der Aufnahmeraum (116) gegenüber der Außenumgebung abgedichtet ist, wodurch ein solches Endoskop sterilisiert werden kann, indem Fluid durch den Aufnahmeraum (116) über den Fluideinlass- und den Fluidauslassanschluß (120, 126) durchgeführt wird, und in diesem aufbewahrt werden kann, in einer Lage, in der die erste und die zweite flexible Röhre (14, 16) nach unten hängen, und in einer sterilen Form, die gegenüber dem Eindringen potentiell kontaminierender Microorganismen geschützt ist,
**dadurch gekennzeichnet, dass** der Endoskopbehälter (102) weiterhin eine Bewegungseinrichtung umfasst, die dafür eingerichtet ist, die Trägerfläche (240; 242) während eines Sterilisationsvorgangs zu bewegen, um dabei Okklusionen zu verringern, und **dadurch, dass** die Trägerfläche (242) zum Drehen eingerichtet ist.

2. Endoskopbehälter nach Anspruch 1, der weiterhin eine Einrichtung umfasst, die dafür eingerichtet ist, einen Flüssigkeitsstrom unter Druck und dabei eine Drehung der Trägerfläche (240; 242) hervorzurufen.

3. Endoskopbehälter nach Anspruch 1 oder 2, bei welchem der Behälter (102) weiterhin eine Abfolge von Anschlüssen (136) umfasst, um einem oder mehreren Lumen in dem Endoskop (10) Fluid zuzuleiten.

4. Endoskopbehälter nach einem der vorhergehenden Ansprüche, der weiterhin eine klappbare Tür (154), die in den Aufnahmeraum (116) führt, um **dadurch** ein solches Endoskop (10) einzuführen, und eine Dichtung (160) um diese herum umfasst.

5. Endoskopbehälter nach Anspruch 4, der weiterhin eine zweite klappbare Tür (154) an einer gegenüberliegenden Seite des Behälters, die in den Aufnahmeraum (116) führt, um **dadurch** das Endoskop (10) einzuführen, und eine Dichtung (160) um diese herum umfasst.

6. Endoskopbehälter nach einem der vorhergehenden Ansprüche, der weiterhin Rollen (108) an dem unteren Teil von diesem umfasst, um ein Bewegen des Behälters (102) zu unterstützen.

7. Endoskopbehälter nach einem der vorhergehenden Ansprüche, bei welchem der Aufnahmeraum (116) ausreichend lang ist, um ein Endoskop mit einer ersten flexiblen Röhre (14) auszunehmen, welche eine Länge von einem Meter aufweist.

8. Endoskopbehälter nach Anspruch 7, bei welchem der Aufnahmeraum (116) ausreichend lang ist, um ein Endoskop mit einer ersten flexiblen Röhre (14) aufzunehmen, welche eine Länge von zwei Metern aufweist.

9. Endoskopbehälter (102) nach einem der vorhergehenden Ansprüche, in Kombination mit einem Endoskop (10), welches einen Körper (12) und eine erste und eine zweite flexible Röhre (14, 16), die an dem Körper angebracht sind, aufweist, wobei der Aufnahmeraum (116) so bemessen und geformt ist, dass er sich eng an die Größe und die Form des Endoskops (10) anfügt.

## Revendications

1. Enceinte d'endoscope (102) destinée à stériliser un endoscope (10) présentant un corps (12), et des premier et second tubes souples (14, 16) fixés au corps, l'enceinte d'endoscope comprenant :
➢ un espace de réception (116) apte à recevoir un endoscope (10) avec une orientation par rapport au premier tube souple (14) dépendant verticalement vers le bas du corps d'endoscope (12), dans lequel l'espace de réception (116) comprend un premier espace dépendant vers le bas (118) apte à recevoir le premier tube souple (14) de l'endoscope dépendant vers le bas, et un second espace dépendant vers le bas (120), séparé du premier espace dépendant vers le bas (118) par une surface de support (240), et apte à recevoir le second
tube souple (16) de l'endoscope dépendant vers le bas ;
➢ un connecteur de fluide d'entrée (122), en communication de fluide avec l'espace de réception (116) et qui présente une fermeture destinée à isoler l'espace de réception d'un environnement extérieur à travers le connecteur de fluide d'entrée ; et
➢ un connecteur de fluide de sortie (126), en communication de fluide avec l'espace de réception (116) et qui présente une fermeture destinée à isoler l'espace de réception de l'environnement extérieur à travers le connecteur de fluide de sortie ;
l'espace de réception (116) étant étanchéifié vis-à-vis de l'environnement extérieur grâce à quoi il est possible de stériliser un endoscope en faisant passer un fluide à travers l'espace de réception (116) par l'intermédiaire des connecteurs de fluide d'entrée et de sortie (122, 126), et de le stocker à l'intérieur avec une orientation par rapport aux premier et second tubes souples (14, 16) dépendant vers le bas, et sous une forme stérile protégée contre une entrée de micro-organismes potentiellement contaminants,
**caractérisée en ce que** l'enceinte d'endoscope (102) comprend en outre des moyens de déplacement aptes à déplacer la surface de support (240 ; 242) au cours d'un procédé de stérilisation de manière à réduire de ce fait des occlusions, et **en ce que** la surface de support (242) est apte à tourner.

2. Enceinte d'endoscope selon la revendication 1, comprenant en outre des moyens aptes à provoquer la circulation d'un liquide sous pression et à faire tourner de ce fait la surface de support (240 ; 242).

3. Enceinte d'endoscope selon la revendication 1 ou la revendication 2, dans laquelle l'enceinte (102) comprend en outre une série de raccordements (136) destinés à approvisionner en liquide une ou plusieurs lumières situées dans l'endoscope (10).

4. Enceinte d'endoscope selon l'une quelconque des revendications précédentes, comprenant en outre une porte articulée (154) qui permet d'accéder à l'espace de réception (116), grâce à quoi il est possible d'insérer par là un endoscope (10) et un joint (160).

5. Enceinte d'endoscope selon la revendication 4, comprenant en outre une seconde porte articulée (154) située sur un côté opposé de l'enceinte et qui permet d'accéder à l'espace de réception (116), grâce à quoi il est possible d'insérer par là l'endoscope (10) et un joint (160).

6. Enceinte d'endoscope selon l'une quelconque des revendications précédentes, comprenant en outre des roulettes (108) situées sur une partie inférieure de celle-ci afin de faciliter le déplacement de l'enceinte (102).

7. Enceinte d'endoscope selon l'une quelconque des revendications précédentes, dans laquelle l'espace de réception (116) est suffisamment long pour recevoir un endoscope avec un premier tube souple (14) qui présente une longueur de un mètre.

8. Enceinte d'endoscope selon la revendication 7, dans laquelle l'espace de réception (116) est suffisamment long pour recevoir un endoscope avec un premier tube souple (14) qui présente une longueur de deux mètres.

9. Enceinte d'endoscope (102) selon l'une quelconque des revendications précédentes, en association avec un endoscope (10) qui présente un corps (12), et des premier et second tubes souples (14, 16) fixés au corps, dans laquelle l'espace de réception (116) est dimensionné et mis en forme de manière à s'adapter étroitement à la taille et à la forme de l'endoscope (10).
